# EUROPEAN PATENT SPECIFICATION

(11) **EP 2 698 373 B1**
(45) Date of publication and mention of the grant of the patent: **19.09.2018**
(21) Application number: 13180256.3
(22) Date of filing: 13.08.2013
(51) Int. Cl.: C07F 9/90, C07F 9/94, C07C 395/00, C23C 16/18

(54) **Precursors for gst films in ald/cvd processes**
Vorläufer für GST-Filme in ALD/CVD-Verfahren
Précurseurs de films gst dans un processus cvd/ald

(30) Priority: 13.08.2012 US 201213572973
(43) Date of publication of application: 19.02.2014
(60) Divisional application: 17190072.3
(73) Proprietor: Versum Materials US, LLC, Tempe, AZ 85284 (US)
(72) Inventor: Xiao, Manchao, San Diego, CA 92130 (US); Buchanan, Iain, Stirling FK7 9LB (GB); Lei, Xinjian, Vista, CA 92081 (US)
(74) Representative: Beck Greener

(56) References cited:
- EP-A1- 2 083 096
- EP-A1- 2 444 405
- WO-A1-2011/027321
- WO-A1-2013/048186
- WO-A2-2011/056519
- US-A1- 2012 021 590
- US-A1- 2012 028 410
- N.S. VYAZANKIN ET AL: "Tris(triethylsilyl)antimony and related compounds", JOURNAL OF ORGANOMETALLIC CHEMISTRY, vol. 6, no. 5, 1 November 1966 (1966-11-01), pages 474-483, XP055084638, ISSN: 0022-328X, DOI: 10.1016/S0022-328X(00)80201-6
- DATABASE CA [Online] CHEMICAL ABSTRACTS SERVICE, COLUMBUS, OHIO, US; VYAZANKIN, N. S. ET AL: "Synthesis and properties of tris(triphenylsilyl)antimony and related compounds", XP002715444, retrieved from STN Database accession no. 1970:31946 & VYAZANKIN, N. S. ET AL: "Synthesis and properties of tris(triphenylsilyl)antimony and related compounds", ZHURNAL OBSHCHEI KHIMII , 39(9), 2005-11 CODEN: ZOKHA4; ISSN: 0044-460X, 1969,
- BECKER GERD ET AL: "Trimethylsilyl derivatives of VB-elements. III. Tris(trimethylsilyl)- and tris(trimethylstannyl)bismuthine as well as tetrakis(trimethylsilyl)dibismuthine", ZEITSCHRIFT FUER NATURFORSCHUNG. TEIL B, ANORGANISCHE CHEMIE, ORGANISCHE CHEMIE, VERLAG DER ZEITSCHRIFT FUER NATURFORSCHUNG, TUEBINGEN, DE, vol. 37B, no. 1, 1 January 1982 (1982-01-01), pages 91-96, XP008165501, ISSN: 0340-5087
- KIRILL YU. MONAKHOV ET AL: "Reduction vs. Metathesis in the Reactions of Bismuth Tribromide with a Bulky Lithium Silanide â?? An Experimental and Theoretical Study", EUROPEAN JOURNAL OF INORGANIC CHEMISTRY, vol. 2010, no. 2, 1 January 2010 (2010-01-01), pages 322-332, XP055084752, ISSN: 1434-1948, DOI: 10.1002/ejic.200900773

## Description

### BACKGROUND OF THE INVENTION

As an emerging technology, phase change materials attract more and more interest for their applications in manufacturing a new type of highly integrated, nonvolatile, memory devices: phase change random access memory (PRAM). Phase change random acess memory (PRAM) devices are synthesized using materials that undergo a reversible phase change between crystalline and amorphous phases, that have distinctly different resistances. The most commonly used phase change materials are ternary compositions of chalcogenide of group 14 and group 15 elements, such as germanium-antimony-tellurium compounds, commonly abbreviated as GST.

One of the technical hurdles in designing a PRAM cell is that in order to overcome the heat dissipation during the switching of GST materials from crystalline to amorphous states at certain temperatures, a high level of reset current has to be applied. This heat dissipation can be greatly reduced by confining the GST material into contact plugs, which in turn reduces the reset current needed. To build GST plugs on the substrate, atomic layer deposition (ALD) processes are used to produce films with high conformality and chemical composition uniformity.

Relevant prior art includes:
Sang-Wook Kim, S. Sujith, Bun Yeoul Lee, Chem. Commun., 2006, pp 4811-4813;
Stephan Schulz, Martin Nieger, J. Organometallic Chem., 570, 1998, pp 275-278;
Byung Joon Choi, et al. Chem Mater. 2007, 19, pp 4387-4389 ; Byung Joon Choi, et al. J. Electrochem. Soc., 154, pp H318-H324 (2007);
Ranyoung Kim, Hogi Kim, Soongil Yoon, Applied Phys. Letters, 89, pp 102-107 (2006);
Junghyun Lee, Sangjoon Choi, Changsoo Lee, Yoonho Kang, Daeil Kim, Applied Surface Science, 253 (2007) pp 3969-3976;
G. Becker, H. Freudenblum, O. Mundt, M. reti, M. Sachs, Synthetic Methods of Organometallic and Inorganic Chemistry, vol. 3, H.H. Karsch, New York, 1996, p. 193;
Sladek, A., Schmidbaur, H., Chem. Ber. 1995, 128, pp 565-567;
US patents and patent applications:
   US 2006/0049447 A1;
   US 2006/0039192 A1;
   US 2006/0072370 A1;
   US 2006/0172083 A1;
   US 8,148,197;
   US 2012/171812 A1;
   US 7,817,464; and
   US2012/028410 discloses ALD deposition of GeSbTe films using reactive and co-reactive precursors for each metal.

### BRIEF SUMMARY OF THE INVENTION

In one aspect, the present invention provides an ALD or CVD process for making a germanium-antimony-tellurium (GST) or germanium-bismuth-tellurium (GBT) film on a surface of a substrate, the process comprising the steps of: introducing into a deposition chamber a germanium precursor selected from Ge(OR¹⁴)₄₋ₓLₓ, wherein L is selected from Cl, Br, I, or mixtures thereof; x is 0, 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; introducing into the deposition chamber a silyltellurium precursor selected from:
(a) 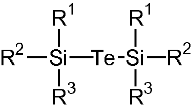
(b) 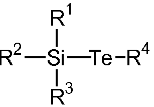
(c) 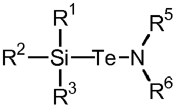
where R¹, R², R³, R⁴, R⁵, and R⁶ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; introducing into the deposition chamber a germanium precursor as defined above; introducing into the deposition chamber a silylantimony or silylbismuth precursor selected from:
(A) 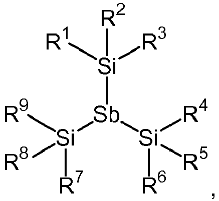
(B) 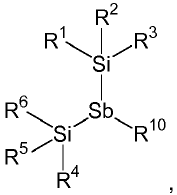
(C) 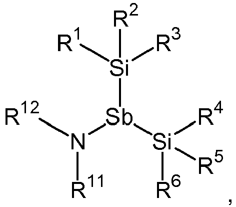 and (D) tris(trimethylsilyl)bismuth, tris(triethylsilyl)bismuth or tris(tert-butyldimethylsilyl)bismuth; where R¹⁻¹⁰ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are independently selected from a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group, to form a silylantimony or silylbismuth monolayer; and repeating the steps above until a desired thickness is reached; wherein the term "alkyl" denotes a linear or branched functional group having from 1 to 10 carbon atoms and optionally including one or more functional groups selected from an alkoxy group and a dialkylamino group attached thereto.

Preferably the germanium precursor is selected from Ge(OMe)₄, Ge(OEt)₄, Ge(OPrⁿ)₄, Ge(OPrⁱ)₄, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl(OPrⁿ)₃, GeCl₂(OPrⁿ))₂, GeCl₂(OPrⁱ)₂, GeCl₃(OPrⁱ), GeCl(OBu^{t})₃, GeCl₂(OBu^{t}))₂, and GeCl₃(OBu^{t}).

Preferably the silylantimony precursor is selected from tris(trimethylsilyl)antimony, tris(triethylsilyl)antimony, tris(tert-butyldimethylsilyl)antimony, and tris(dimethylsilyl)antimony.

Preferably the disilyltellurium precursor is selected from the group consisting of bis(trimethylsilyl)tellurium, bis(triethylsilyl)tellurium, and bis(tert-butyldimethylsilyl)tellurium.

Preferably the steps are repeated in sequence.

Preferably the temperature of the deposition chamber is from room temperature to 400°C.

Features described in connection with one aspect of the invention can be used in connection with any other aspect of the invention.

### DETAILED DESCRIPTION OF THE INVENTION

GST or GBT (germanium-bismuth-tellurium) materials in PRAM devices are normally deposited in the temperature range of 180° - 300° C. It was found that films deposited at 200° C have the best chemical and structural properties. The ALD process requires precursors with high chemical reactivity and reaction selectivity. Currently existing precursors, such as dialkyltellurium, trialkylantimony, and alkylgermanes do not have the required reactivity at given deposition conditions to be used in ALD cycles. Frequently, plasma is used to promote the deposition.

The present invention makes use of silylantimony or silylbismuth precursors, which generate antimony or bismuth layers in an ALD or CVD process. The antimony or bismuth layer may react with subsequently deposited germanium and tellurium layers in a plurality of ALD cycles to form GST or GBT ternary material films, which are suitable for PRAM devices. In certain embodiments, this invention discloses several silylantimony precursors with high reactivity and thermal stability, and the chemistries to be used in an ALD process to deposit a GST or GBT film in conjunction with other chemicals.

Exemplary alkyl groups include, but are not limited to, methyl, ethyl, propyl, isopropyl, butyl, isobutyl, sec-butyl, tert-butyl, pentyl, iso-pentyl, tert-pentyl, hexyl, iso-hexyl, and neo-hexyl. The term "cyclic alkyl" denotes a cyclic functional group having from 3 to 10 or from 4 to 10 carbon atoms or from 5 to 10 carbon atoms. Exemplary cyclic alkyl groups include, but are not limited to, cyclobutyl, cyclopentyl, cyclohexyl, and cyclooctyl groups. The term "aromatic" denotes an aromatic cyclic functional group having from 4 to 10 carbon atoms or from 6 to 10 carbon atoms. Exemplary aryl groups include, but are not limited to, phenyl, benzyl, chlorobenzyl, tolyl, and o-xylyl. The term "alkenyl group" denotes a group which has one or more carbon-carbon double bonds and has from 2 to 10 or from 2 to 6 or from 2 to 4 carbon atoms.

An ALD reaction can be illustrated by the following two schemes for deposting Ge-Te-Ge-Sb (first scheme) or Ge-Te-Ge-Bi (second scheme) films:

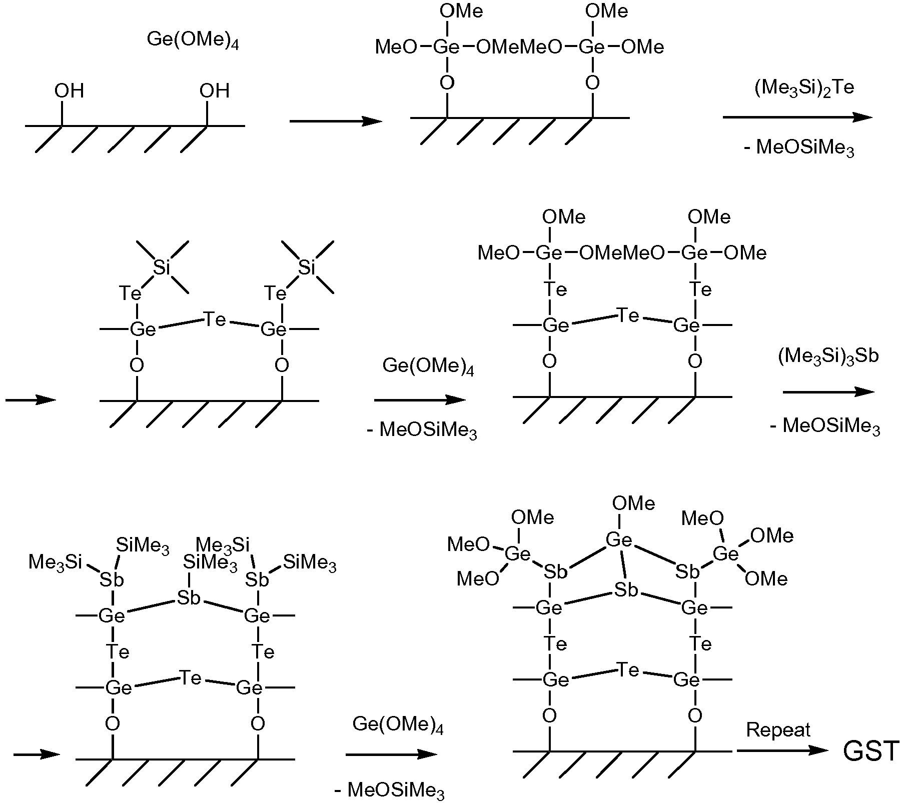

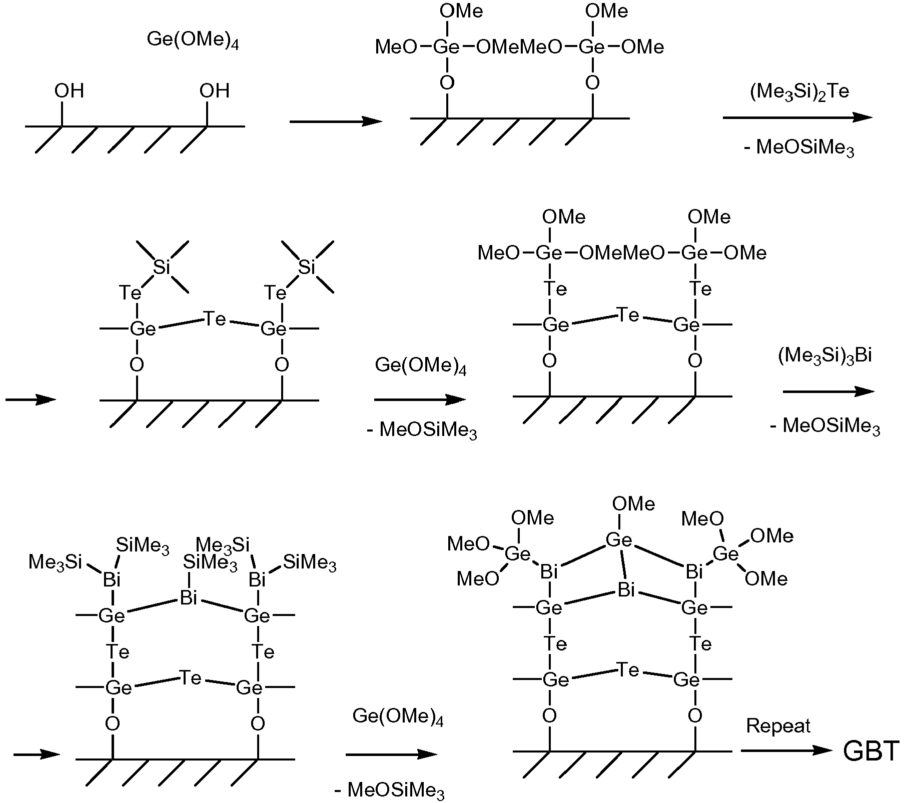

Step 1. Tetrakis(methoxy)germane is introduced and forms a molecular layer of alkoxygermane on the surface of the substrate.

Step 2. Hexamethyldisilyltellurium reacts with alkoxygermane layer to form Te-Ge bonds with elimination of methoxytrimethylsilane. A Te layer with silyl substituents is formed.

Step 3. Tetrakis(methoxy)germane reacts with remaining silyl groups on the layer to form Te-Ge bonds with elimination of methoxytrimethylsilane. A Ge layer with methoxy substituents is formed.

Step 4. Tris(trimethylsilyl)antimony or tris(trimethylsilyl)bismuth is introduced to form an antimony or bismuth layer with silyl substituents on the top of the germanium layer via elimination of methoxytrimethylsilane.

Step 5. Tetrakis(methoxy)germane reacts with remaining silyl groups on the Sb or Bi layer to form Sb-Ge or Bi-Ge bonds, generating a Ge layer with methoxy substituents.

An ALD cycle is then repeated, potentially many times, until the desired film thickness is achieved. The next cycle starts with step 1, again, etc. In another embodiment, step 2 and step 4 can be switched, e.g. if Ge-Sb-Ge-Te or Ge-Bi-Ge-Te films are to be deposited.

Examples of germanium compound having both halide and alkoxy ligand include, for example, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl₂(OPrⁿ))₂, GeCl₃(OPrⁿ), GeCl(OPrⁱ)₃, GeCl₂(OPrⁱ))₂, GeCl₃(OPrⁱ), GeCl(OBu^{t})₃, GeCl₂(OBu^{t}))₂, and GeCl₃(OBu^{t}), wherein OBu^{t} is tert-butyl alkoxy, OPrⁿ is n-propoxy , and OPrⁱ is iso-propoxy. Such compounds are preferably thermally stable and have bulky alkoxy groups which prevents disproportionation reactions.

The silyltellurium precursors can include disilyltellurium, silylalkyltellurium, or silylaminotellurium as described above.

### EXAMPLES

### Example 1: Synthesis of Tris(trimethylsilyl)antimony

1.22g (0.01 mol) of 200 mesh antimony powder, 0.72 g (0.03 mol) of lithium hydride, and 40 ml of tetrahydrofuran (THF) were placed in a 100 ml flask. With stirring, the mixture was refluxed for 4 hours. All of the black powder constituting antimony disappeared, and a muddy colored precipitate was formed. Then, the mixture was cooled down to - 20 °C; 3.3 g (0.03 mol) of trimethylchlorosilane was added. The mixture was allowed to warm up to room temperature. After stirring for 4 hours, the mixture was filtered under inert atmosphere. The solvent was removed by distillation. Tris(trimethylsilyl)antimony was purified by vacuum distillation.

### Example 2: Synthesis of Tris(dimethylsilyl)antimony

1.22g (0.01 mol) of 200 mesh antimony powder, 0.72 g (0.03 mol) of lithium hydride, and 40 ml of tetrahydrofuran (THF) were placed in a 100 ml flask. With stirring, the mixture was refluxed for 4 hours. All of the black powder constituting antimony disappeared, and a muddy colored precipitate was formed. Then, the mixture was cooled down to - 20 °C; 2.83 g (0.03 mol) of dimethylchlorosilane was added. The mixture was allowed to warm up to room temperature. After stirring for 4 hours, the mixture was filtered under inert atmosphere. The solvent was removed by distillation. Tris(dimethylsilyl)antimony was purified by vacuum distillation.

### Example 3: Synthesis of Tris(dimethylsilyl)antimony

3.65 g (0.03 mol) of 200 mesh antimony powder, 2.07 g (0.09 mol) of sodium, 1.15g (0.009 mol) of naphthalene, and 50 ml of THF were placed in a 100 ml flask. The mixture was stirred at room temperature for 24 hours. All of the black powder constituting antimony and sodium disappeared, and a muddy colored precipitate was formed. Then, the mixture was cooled down to - 20°C; 8.51 g (0.09 mol) of dimethylchlorosilane was added. The mixture was allowed to warm up to room temperature. After stirring for 4 hours, the mixture was filtered under inert atmosphere. The solvent was removed by distillation. Tris(dimethylsilyl)antimony was purified by vacuum distillation.

### Example 4: Synthesis of Tris(trimethylsilyl)bismuth (Prophetic)

6.27 g (0.03 mol) of 200 mesh bismuth powder, 2.07 g (0.09 mol) of sodium, 1.15g (0.009 mol) of naphthalene, and 50 ml of THF is placed in a 100 ml flask. The mixture is stirred at room temperature for 24 hours. All of the black powder constituting bismuth and sodium disappears, and a muddy colored precipitate forms. Then, the mixture is cooled down to - 20 °C; 9.77 g (0.09 mol) of trimethylchlorosilane is added. The mixture is allowed to warm up to room temperature. After stirring for 4 hours, the mixture is filtered under inert atmosphere. The solvent is removed by distillation. Tris(trmethylsilyl)bismuth can be purified by vacuum distillation.

### Example 5: Deposition of GeSbTe Films in ALD Reactor

Deposition of GeSbTe film using atomic layer deposition (ALD) technique including the following steps:
a) Substrates on which films are to be deposited are loaded to an ALD reactor;
b) The reactor is flashed with N₂ and pumped down to low pressure of less than 1 torr (130 Pa) and heated up to a temperature at which film deposition is performed;
c) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂; and
d) A fixed flow rate of the vapor of disilyltellurium compound as Te precursor is introduced to the reactor. The reactor is saturated with this vapor for a short fixed time (typical less than 5 seconds), and then pumped down to 1 torr (130 Pa), followed by flashing with N₂;
e) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂;
f) A fixed flow rate of the vapor of trisilylantimony compound as Sb precursor is introduced to the reactor. The reactor is saturated with this vapor for a short fixed time (typical less than 5 seconds), and then pumped down to 1 torr (130 Pa), followed by flashing with N₂; and

steps c) to f) are repeated until a desired thickness of the film is achieved.

With the deposition chemistry, highly conformal GeSbTe films can be deposited on the surface of substrate materials such as silicon, silicon oxide, silicon nitride, titanium nitride. The process temperature range could be from room temperature to 400°C.

### Example 6: Deposition of GeBiTe Films in ALD Reactor (prophetic)

Deposition of GeBiTe film using atomic layer deposition (ALD) technique including the following steps:
a) Substrates on which films are to be deposited are loaded to an ALD reactor;
b) The reactor is flashed with N₂ and pumped down to low pressure of less than 1 torr (130 Pa) and heated up to a temperature at which film deposition is performed;
c) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂; and
d) A fixed flow rate of the vapor of disilyltellurium compound as Te precursor is introduced to the reactor. The reactor is saturated with this vapor for a short fixed time (typical less than 5 seconds), and then pumped down to 1 torr (130 Pa), followed by flashing with N₂;
e) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂;
f) A fixed flow rate of the vapor of trisilylbismuth compound as Bi precursor is introduced to the reactor. The reactor is saturated with this vapor for a short fixed time (typical less than 5 seconds), and then pumped down to 1 torr (130 Pa), followed by flashing with N₂; and

steps c) to f) are repeated until a desired thickness of the film is achieved.

With the deposition chemistry, highly conformal GeBiTe films can be deposited on the surface of substrate materials such as silicon, silicon oxide, silicon nitride, titanium nitride. The process temperature range could be from room temperature to 400°C.

## Claims

1. An ALD or CVD process for making a germanium-antimony-tellurium (GST) or germanium-bismuth-tellurium (GBT) film on a surface of a substrate, the process comprising the steps of:
introducing into a deposition chamber a germanium precursor selected from Ge(OR¹⁴)₄₋ₓLₓ, wherein L is selected from Cl, Br, I, or mixtures thereof; x is 0, 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group;
introducing into the deposition chamber a silyltellurium precursor selected from:
(a) 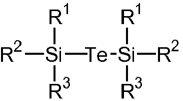
(b) 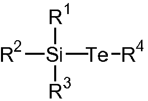
(c) 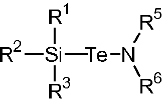 where R¹, R², R³, R⁴, R⁵, and R⁶ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group;
introducing into the deposition chamber a germanium precursor as defined above;
introducing into the deposition chamber a silylantimony or silylbismuth precursor selected from:
(A) 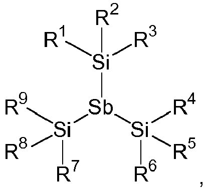
(B) 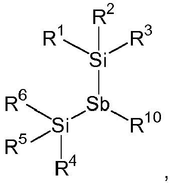
(C) 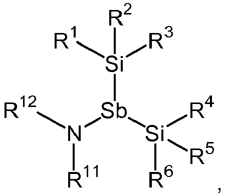
and (D) tris(trimethylsilyl)bismuth, tris(triethylsilyl)bismuth or tris(tert-butyldimethylsilyl)bismuth;
where R¹⁻¹⁰ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are independently selected from a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group, to form a silylantimony or silylbismuth monolayer; and
repeating the steps above until a desired thickness is reached;
wherein the term "alkyl" denotes a linear or branched functional group having from 1 to 10 carbon atoms and optionally including one or more functional groups selected from an alkoxy group and a dialkylamino group attached thereto.

2. The process of claim 1 wherein the germanium precursor is selected from Ge(OMe)₄, Ge(OEt)₄, Ge(OPrⁿ)₄, Ge(OPrⁱ)₄, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl(OPrⁿ)₃, GeCl₂(OPrⁿ))₂, GeCl₂( OPrⁱ)₂, GeCl₃(OPrⁱ), GeCl(OBu^{t})₃, GeCl₂(OBu^{t}))₂, and GeCl₃(OBu^{t}).

3. The process of either one of the preceding claims wherein the silylantimony precursor is selected from tris(trimethylsilyl)antimony, tris(triethylsilyl)antimony, tris(tertbutyldimethylsilyl)antimony, and tris(dimethylsilyl)antimony.

4. The process of any one of Claims 1 to 3 wherein the disilyltellurium precursor is selected from the group consisting of bis(trimethylsilyl)tellurium, bis(triethylsilyl)tellurium, and bis(tert-butyldimethylsilyl)tellurium.

5. The process of any one of the preceding claims wherein the steps are repeated in sequence.

6. The process of any one of the preceding claims wherein the temperature of the deposition chamber is from room temperature to 400°C.

## Patentansprüche

1. ALD- oder CVD-Verfahren zur Herstellung eines Germanium-Antimon-Tellur-(GST-) oder Germanium-Wismut-Tellur-(GBT-)Films auf einer Oberfläche eines Substrats, wobei das Verfahren die folgenden Schritte umfasst:
Einführen eines Germaniumvorläufers, ausgewählt aus Ge(OR¹⁴)₄₋ₓLₓ, in eine Abscheidungskammer, wobei L aus Cl, Br, I oder Mischungen davon ausgewählt ist; x 0, 1, 2 oder 3 ist; R¹⁴ eine C₁-C₁₀-Alkylgruppe, eine C₂-C₁₀-Alkenylgruppe, eine cyclische C₃-C₁₀-Alkylgruppe, eine cyclische C₃-C₁₀-Alkenylgruppe oder eine aromatische C₄-C₁₀-Gruppe ist;
Einführen eines Silyltellurvorläufers in die Abscheidungskammer, ausgewählt aus:
(a) 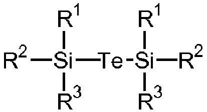
(b) 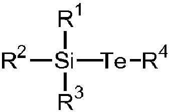
(c) 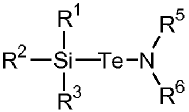 wobei R¹, R², R³, R⁴, R⁵ und R⁶ unabhängig aus Wasserstoff, einer C₁-C₁₀-Alkylgruppe, einer C₂-C₁₀-Alkenylgruppe, einer cyclischen C₃-C₁₀-Alkylgruppe, einer cyclischen C₃-C₁₀-Alkenylgruppe oder einer aromatischen C₄-C₁₀-Gruppe ausgewählt sind;
Einführen eines Germaniumvorläufers wie vorstehend definiert in die Abscheidungskammer;
Einführen eines Silylantimon- oder Silylwismutvorläufers in die Abscheidungskammer, ausgewählt aus:
(A) 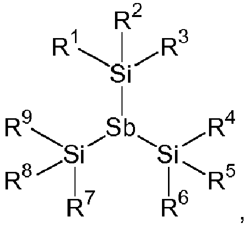
(B) 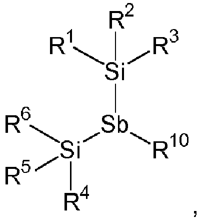
(C) 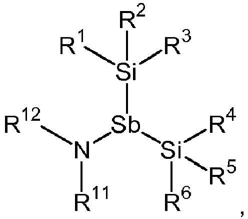
und (D) tris(Trimethylsilyl)wismut, tris(Triethylsilyl)wismut oder tris(Tertbutyldimethylsilyl)wismut;
wobei R¹⁻¹⁰ unabhängig aus Wasserstoff, einer C₁-C₁₀-Alkylgruppe, einer C₂-C₁₀-Alkenylgruppe, einer cyclischen C₃-C₁₀-Alkylgruppe, einer cyclischen C₃-C₁₀-Alkenylgruppe oder einer aromatischen C₄-C₁₀-Gruppe ausgewählt sind; R¹¹ und R¹² unabhängig aus einer C₁-C₁₀-Alkylgruppe, einer C₂-C₁₀-Alkenylgruppe, einer cyclischen C₃-C₁₀-Alkylgruppe, einer cyclischen C₃-C₁₀-Alkenylgruppe oder einer aromatischen C₄-C₁₀-Gruppe ausgewählt sind, um eine Silylantimon- oder Silylwismut-Monoschicht zu bilden; und
Wiederholen der vorstehenden Schritte, bis eine gewünschte Dicke erreicht ist;
wobei der Begriff "Alkyl" eine lineare oder verzweigte Funktionsgruppe bezeichnet, die 1 bis 10 Kohlenstoffatome aufweist und optional eine oder mehrere Funktionsgruppen beinhaltend, die aus einer Alkoxygruppe und einer Dialkylaminogruppe, die daran angehängt sind, ausgewählt sind.

2. Verfahren nach Anspruch 1, wobei der Germaniumvorläufer aus Ge(OMe)₄, Ge(OEt)₄, Ge(OPrⁿ)₄, Ge(OPrⁱ)₄, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl(OPrⁿ)₃, GeCl₂(OPrⁿ))₂, GeCl₂(OPrⁱ)₂, GeCl₃(OPrⁱ), GeCl(OBu^{t})₃, GeCl₂(OBu^{t}))₂ und GeCl₃(OBu^{t}) ausgewählt ist.

3. Verfahren nach einem der vorhergehenden Ansprüche, wobei der Silylantimonvorläufer aus tris(Trimethylsilyl)antimon, tris(Triethylsilyl)antimon, tris(Tertbutyldimethylsilyl)antimon und tris(Dimethylsilyl)antimon ausgewählt ist.

4. Verfahren nach einem der Ansprüche 1 bis 3, wobei der Disilyltellurvorläufer aus der Gruppe ausgewählt ist, die aus bis(Trimethylsilyl)tellur, bis(Triethylsilyl)tellur und bis(Tertbutyldimethylsilyl)tellur ausgewählt ist.

5. Verfahren nach einem der vorhergehenden Ansprüche, wobei die Schritte in Sequenz wiederholt werden.

6. Verfahren nach einem der vorhergehenden Ansprüche, wobei die Temperatur der Abscheidungskammer von Raumtemperatur bis 400 °C reicht.

## Revendications

1. Procédé ALD ou CVD pour la fabrication d'un film de germanium-antimoine-tellure (GST) ou de germanium-bismuth-tellure (GBT) sur une surface d'un substrat, le procédé comprenant les étapes qui consistent à :
introduire dans une chambre de déposition un précurseur de germanium sélectionné à partir de Ge(OR¹⁴)₄₋ₓLₓ, dans lequel L est sélectionné parmi les atomes Cl, Br, I ou des mélanges de ceux-ci ; x vaut 0, 1, 2 ou 3 ; R¹⁴ est un groupe alkyle de C₁ à C₁₀, un groupe alcényle de C₂ à C₁₀,
un groupe alkyle cyclique de C₃ à C₁₀, un groupe alcényle cyclique de C₃ à C₁₀ ou un groupe aromatique de C₄ à C₁₀ ;
introduire dans la chambre de déposition un précurseur de silyltellure sélectionné parmi :
(a) 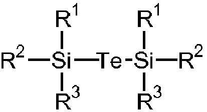
(b) 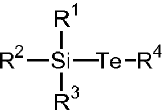
(c) 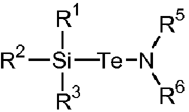 dans lesquels R¹, R², R³, R⁴, R⁵ et R⁶ sont indépendamment sélectionnés parmi un atome hydrogène, un groupe alkyle de C₁ à C₁₀, un groupe alcényle de C₂ à C₁₀, un groupe alkyle cyclique de C₃ à C₁₀, un groupe alcényle cyclique de C₃ à C₁₀ ou un groupe aromatique de C₄ à C₁₀ ;
introduire dans la chambre de déposition un précurseur de germanium tel que défini ci-dessus ;
introduire dans la chambre de déposition un précurseur de silylantimoine ou de silylbismuth sélectionné parmi :
(A) 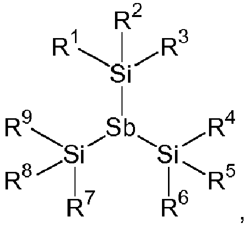
(B) 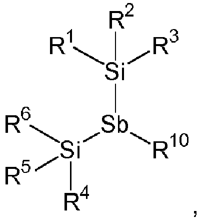
(C) 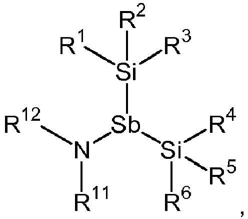
et (D) le tris(triméthylsilyl)bismuth, le tris(triéthylsilyl)bismuth ou le tris(tert-butyldiméthylsilyl)bismuth ; dans lesquels R^{1 à 10} sont indépendamment choisis parmi un atome hydrogène, un groupe alkyle de C₁ à C₁₀, un groupe alcényle de C₂ à C₁₀, un groupe alkyle cyclique de C₃ à C₁₀, un groupe alcényle cyclique de C₃ à C₁₀ ou un groupe aromatique de C₄ à C₁₀ ; R¹¹ et R¹² sont indépendamment sélectionné parmi un groupe alkyle de C₁ à C₁₀, un groupe alcényle de C₂ à C₁₀, un groupe alkyle cyclique de C₃ à C₁₀, un groupe alcényle cyclique de C₃ à C₁₀ ou un groupe aromatique de C₄ à C₁₀, pour former une monocouche de silylantimoine ou de silylbismuth ; et répéter les étapes ci-dessus jusqu'à l'obtention d'une épaisseur souhaitée ;
dans lesquels le terme « alkyle » dénote un groupe fonctionnel linéaire ou ramifié ayant de 1 à 10 atomes de carbone et comprenant éventuellement un ou plusieurs groupes fonctionnels sélectionnés parmi un groupe alcoxy et un groupe dialkylamino fixé à celui-ci.

2. Procédé selon la revendication 1, dans lequel le précurseur de germanium est sélectionné parmi Ge(OMe)₄, Ge(OEt)₄, Ge(OPrⁿ)₄, Ge(OPrⁱ)₄, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl(OPrⁿ)₃, GeCl₂(OPrⁿ))₂, GeCl₂(OPrⁱ)₂, GeCl₃(OPrⁱ), GeCl(OBu^{t})₃, GeCl₂(OBu^{t}))₂ et GeCl₃(OBu^{t}).

3. Procédé selon l'une quelconque des revendications précédentes, dans lequel le précurseur de silylantimoine est sélectionné parmi le tris(triméthylsilyl)antimoine, le tris(triéthylsilyl)antimoine, le tris(tert- butyldiméthylsilyl)antimoine et le tris(diméthylsilyl)antimoine.

4. Procédé selon l'une quelconque des revendications 1 à 3, dans lequel le précurseur de disilyltellure est sélectionné parmi le bis(triméthylsilyl)tellure, le bis(triéthylsilyl)tellure et le bis(tert-butyldiméthylsilyl)tellure.

5. Procédé selon l'une quelconque des revendications précédentes, dans lequel les étapes sont répétées en séquence.

6. Procédé selon l'une quelconque des revendications précédentes, dans lequel la température de la chambre de déposition varie de la température ambiante à 400 °C.
